# EUROPEAN PATENT APPLICATION

(11) **EP 4 535 006 A1**
(43) Date of publication of application: **09.04.2025**
(21) Application number: 23815675.6
(22) Date of filing: 02.05.2023
(51) Int. Cl.: G01N 35/00, G01N 35/02, G01N 35/10

(54) **INSPECTION DEVICE**

(30) Priority: 03.06.2022 JP 2022091057
(71) Applicant: FUJIFILM Corporation, Tokyo 106-8620 (JP)
(72) Inventor: MORI, Takaaki, Ashigarakami-gun, Kanagawa 258-8538 (JP); DENAWA, Tatsuyuki, Ashigarakami-gun, Kanagawa 258-8538 (JP)
(74) Representative: Dehns Germany Partnerschaft mbB
(86) International application number: PCT/JP2023/017166
(87) International publication number: WO 2023/233914

(57) **Abstract**

An examination apparatus includes a linear transport passage that transports both cartridges of a cartridge for pre-treatment which is used for a pre-treatment performed on a specimen before a measurement process is executed, the pre-treatment being performed by heating the specimen in a state in which the specimen is mixed with a pre-treatment liquid, and which has a cell for pre-treatment accommodating the specimen and the pre-treatment liquid, and the cartridge for measurement, the transport passage being capable of transporting the cartridge for pre-treatment while performing the pre-treatment on the specimen; and a specimen transfer mechanism that transfers the pre-treated specimen in which the pre-treatment is completed, from the cartridge for pre-treatment transported at any position between, in the transport passage, a specimen dispensing position where the specimen is dispensed into the reaction cell of the cartridge for measurement and a detection position where an examination target substance in the specimen is optically detected in a downstream of the transport passage in a transport direction, to the cartridge for measurement disposed at the specimen dispensing position.

## Description

### BACKGROUND OF THE INVENTION

### 1. Field of the Invention

The present invention relates to an examination apparatus.

### 2. Description of the Related Art

An examination apparatus which quantitatively or qualitatively detects an examination target substance in a specimen has been known. Such an examination apparatus mainly utilizes an immunoassay principle, and examples thereof include a chemiluminescent enzyme immunological analysis apparatus and a fluorescence immunological analysis apparatus.

In such an examination apparatus, the examination target substance is optically detected by detecting luminescence or fluorescence based on a label such as an enzyme label and a fluorescent label, which has been imparted to the examination target substance in the specimen by an immunoreaction. An examination apparatus in which a process of imparting the label to such a target substance in a specimen and an optically detecting process of the examination target substance are automated has been proposed.

As the automated examination apparatus, a configuration in which a plurality of reaction containers are arranged on a rotary disk, and respective processing sections such as specimen dispensing, reagent dispensing, and light detection are assigned to predetermined rotational angular positions, and an examination is performed while the rotary disk is rotated once has been known (JP2009-31204A).

In addition, as the automated examination apparatus, an examination apparatus which uses a cartridge including a cell in which a reagent used in one examination is accommodated and a reaction cell in which a specimen is dispensed and which linearly transports the cartridge has also been known (JP1993-40122A (JP-H5-40122A)).

The examination apparatus disclosed in JP1993-40122A (JP-H5-40122A) uses a single-use type cartridge, which is different from the examination apparatus disclosed in JP2009-31204A. In addition, the examination apparatus of JP1993-40122A (JP-H5-40122A) has a linear transport passage as a transport passage for transporting the cartridge, and each processing section related to the measurement process of the examination target substance, such as specimen dispensing, reagent dispensing, and light detection, is disposed on the transport passage, the cartridge is transported along the transport passage, and each process is performed in each processing section.

### SUMMARY OF THE INVENTION

In recent years, in immunoassays, a method of performing pre-treatment such as suppressing activity of a substance which inhibits an immunoreaction in a specimen before the immune reaction has been proposed. Depending on examination item (for example, the examination target substance to be detected), detection accuracy can be improved by performing the pre-treatment. The pre-treatment referred to here is a treatment of heating a specimen in a state of being mixed with a pre-treatment liquid. Such pre-treatment is also performed in the examination apparatus disclosed in JP2009-31204A.

In the pre-treatment, there is an appropriate heating time depending on the examination item, and the appropriate heating time may vary depending on the examination item. It is also conceivable to perform the pre-treatment for the same heating time regardless of the examination item with an average heating time. However, since accuracy can be further increased by setting the heating time suitable for each individual examination item, it is desired to perform the pre-treatment on the specimen at the heating time suitable for each individual examination item.

Different from the examination apparatus disclosed in JP2009-31204A, having a rotary measurement line, the examination apparatus disclosed in JP1993-40122A (JP-H5-40122A) has a measurement line for performing the measurement process while linearly transporting a cartridge. In addition, the examination apparatus disclosed in JP1993-40122A (JP-H5-40122A) uses a single-use type cartridge, which is different from the examination apparatus disclosed in JP2009-31204A. In a case where a cartridge is used, the linear measurement line disclosed in JP1993-40122A (JP-H5-40122A) may be advantageous as compared with the rotary measurement line disclosed in JP2009-31204A in terms of simplification and reduction in size of a transport mechanism for transporting the cartridge and a mechanism for detaching the cartridge from the transport passage.

Therefore, in an examination apparatus having a measurement line for linearly transporting the cartridge as disclosed in JP1993-40122A (JP-H5-40122A), there has been a demand for enabling the pre-treatment to be performed for an appropriate heating time for each examination item while suppressing an increase in size.

An object of the present disclosure is to provide an examination apparatus linearly transporting a cartridge, which is capable of performing a pre-treatment for each examination item at an appropriate heating time while suppressing an increase in size in an examination apparatus that linearly transports a cartridge.

An examination apparatus according to the present disclosure is an examination apparatus for performing a measurement process using a cartridge for measurement, having a reaction cell in which a specimen is dispensed and mixed with a reagent, the examination apparatus including a linear transport passage that transports both cartridges of a cartridge for pre-treatment which is used for a pre-treatment performed on the specimen before the measurement process is executed, the pre-treatment being performed by heating the specimen in a state in which the specimen is mixed with a pre-treatment liquid, and which has a cell for pre-treatment accommodating the specimen and the pre-treatment liquid, and the cartridge for measurement, the transport passage being capable of transporting the cartridge for pre-treatment while performing the pre-treatment on the specimen; and a specimen transfer mechanism that transfers the pre-treated specimen in which the pre-treatment is completed, from the cartridge for pre-treatment transported at any position between, in the transport passage, a specimen dispensing position where the specimen is dispensed into the reaction cell of the cartridge for measurement and a detection position where an examination target substance in the specimen is optically detected in a downstream of the transport passage in a transport direction, to the cartridge for measurement disposed at the specimen dispensing position.

The examination apparatus may include, as the specimen transfer mechanism, a dispensing mechanism that performs suction and discharge of a liquid by a nozzle, and the dispensing mechanism may transfer the pre-treated specimen by suctioning the pre-treated specimen from the cell for pre-treatment of the cartridge for pre-treatment at the any position on the transport passage, and dispensing the pre-treated specimen into the reaction cell of the cartridge for measurement at the specimen dispensing position.

The dispensing mechanism may be also used for dispensing a specimen which has not been subjected to the pre-treatment into the reaction cell, in addition to the transfer of the pre-treated specimen from the cell for pre-treatment to the reaction cell.

In a case where a plurality of the cartridges for measurement and a plurality of the cartridges for pre-treatment, which are used for different specimens, are sequentially transported on the transport passage, the examination apparatus may further include a cover member that is capable of covering the cartridges for measurement and the cartridges for pre-treatment above the transport passage and prevents the specimen transferred by the specimen transfer mechanism from being mixed into the cartridge for measurement and the cartridge for pre-treatment, which are used for another specimen.

The specimen transfer mechanism may include a dispensing mechanism that performs suction and discharge of a liquid by a nozzle and a pre-treatment cartridge moving mechanism that detaches the cartridge for pre-treatment from the transport passage and moves the cartridge for pre-treatment to an upstream side in the transport direction of the transport passage, and after the cartridge for pre-treatment is moved by the pre-treatment cartridge moving mechanism, the pre-treated specimen may be transferred from the cell for pre-treatment to the reaction cell by the dispensing mechanism.

The dispensing mechanism may be also used for dispensing a specimen which has not been subjected to the pre-treatment into the reaction cell, in addition to the transfer of the pre-treated specimen from the cell for pre-treatment to the reaction cell.

According to the technology of the present disclosure, it is possible to provide an examination apparatus linearly transporting a cartridge, which is capable of performing a pre-treatment for each examination item at an appropriate heating time while suppressing an increase in size.

### BRIEF DESCRIPTION OF THE DRAWINGS

Fig. 1 is a schematic diagram showing an overall configuration of an examination apparatus according to a first embodiment.
Figs. 2A and 2B are schematic views of an example of a cartridge for measurement, in which Fig. 2A is a top view of the cartridge and Fig. 2B is a front view of the cartridge.
Fig. 3 is a process diagram showing a procedure of a measurement process based on a chemiluminescent enzyme immunoassay method.
Fig. 4 is a perspective view of a cartridge for pre-treatment and a cartridge for measurement.
Fig. 5 is a plan view schematically showing a configuration of a transport mechanism and a dispensing mechanism.
Fig. 6 is a cross-sectional view taken along a line VI-VI in Fig. 5.
Fig. 7 is a perspective view schematically showing the configuration of the transport mechanism and the dispensing mechanism.
Fig. 8 is a view schematically showing a step of moving the cartridge in a transport direction with movement of a heat block.
Fig. 9 is a perspective view showing a modification example of the examination apparatus.
Fig. 10 is a plan view schematically showing a configuration of a transport passage and a specimen transfer mechanism of an examination apparatus according to a second embodiment.
Fig. 11 is a perspective view schematically showing the configuration of the transport passage and the specimen transfer mechanism of the examination apparatus according to the second embodiment.

### DESCRIPTION OF THE PREFERRED EMBODIMENTS

Hereinafter, an examination apparatus according to the embodiment of the present disclosure will be described with reference to the drawings. Constituent elements indicated by the same reference numeral in the drawings mean the same constituent element. However, unless otherwise specified in the specification, each constituent element is not limited to one and may be plural.

### "Examination apparatus according to first embodiment"

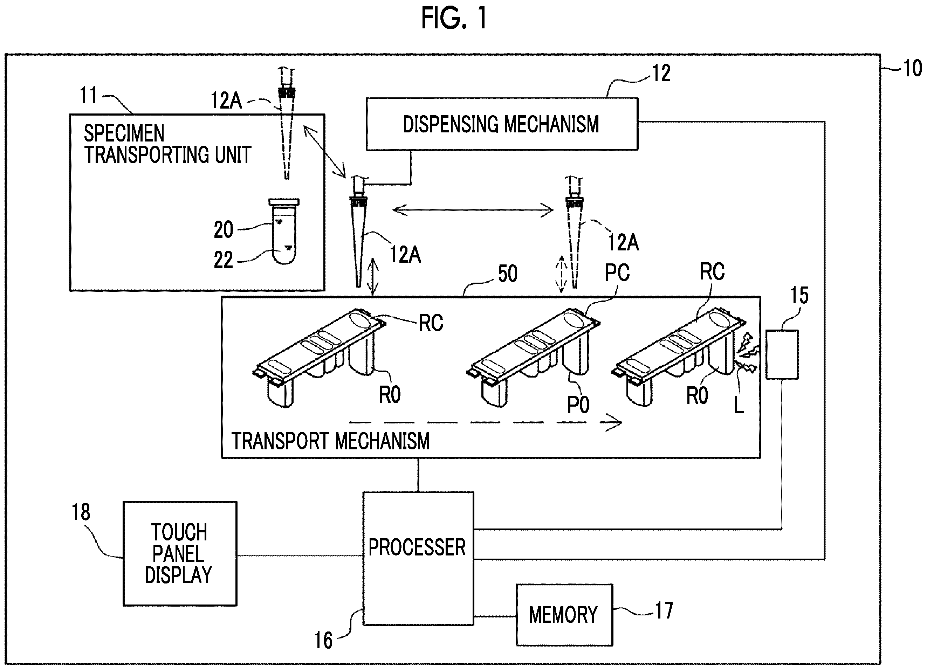

Fig. 1 is a schematic diagram showing an overall configuration of an examination apparatus 10 according to a first embodiment. As an example, the examination apparatus 10 is an immunological analysis apparatus which detects an examination target substance in a specimen collected from a biological body by an antigen-antibody reaction. The examination apparatus 10 optically detects the examination target substance in a specimen using a cartridge RC for measurement, and outputs an examination result.

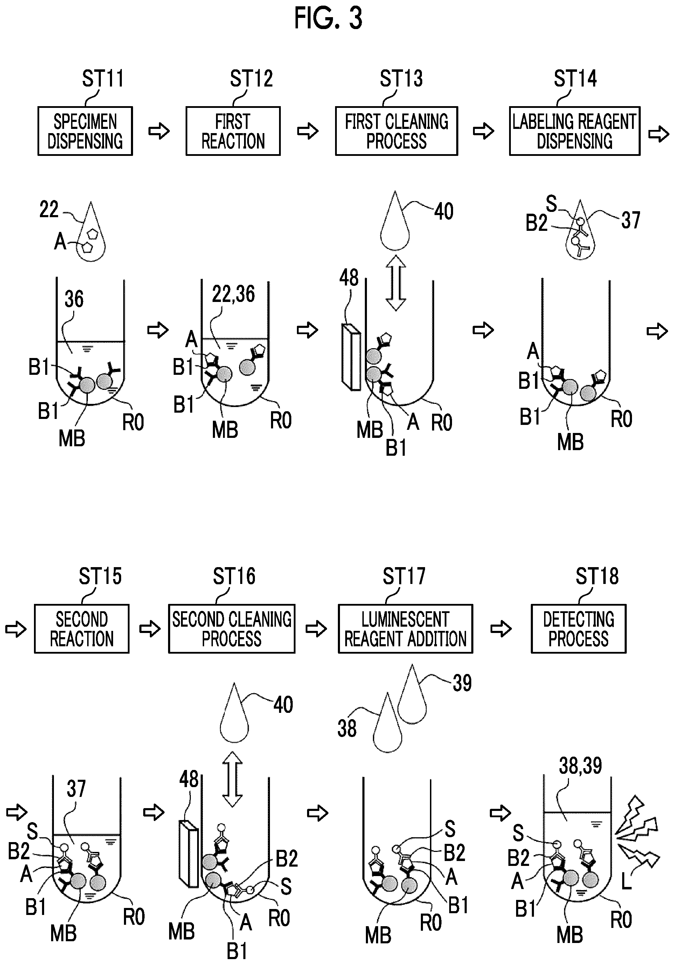

As an example, the specimen 22 is a body fluid such as blood, collected from the biological body. In a case where the specimen 22 is blood, the specimen 22 may be whole blood, blood plasma, serum, or the like. In addition, a centrifuge may be provided in the examination apparatus 10 to extract blood plasma or serum from whole blood. Furthermore, the examination target substance A (see Fig. 3) which can be contained in the specimen 22 is an antigen, an antibody, a protein, a low-molecular-weight compound, or the like. The specimen 22 is not limited to the blood, and may be a substance collected from the biological body, such as urine and body fluid. A specimen collection container 20 which accommodates the specimen 22 to be examined is loaded into the examination apparatus 10 and is subjected to examination. A plurality of the specimen collection containers 20 may be collectively loaded into the examination apparatus 10 such that the examination apparatus 10 can continuously process examinations for the plurality of the specimens 22.

The cartridge RC for measurement is attachably and detachably loaded into the examination apparatus 10. The cartridge RC for measurement is a single-use type used once for one specimen 22. As an example, the cartridge RC for measurement includes all reagent necessary for the examination of the specimen 22.

As an example, the examination apparatus 10 according to the present example performs an examination based on a chemiluminescent enzyme immunoassay method.

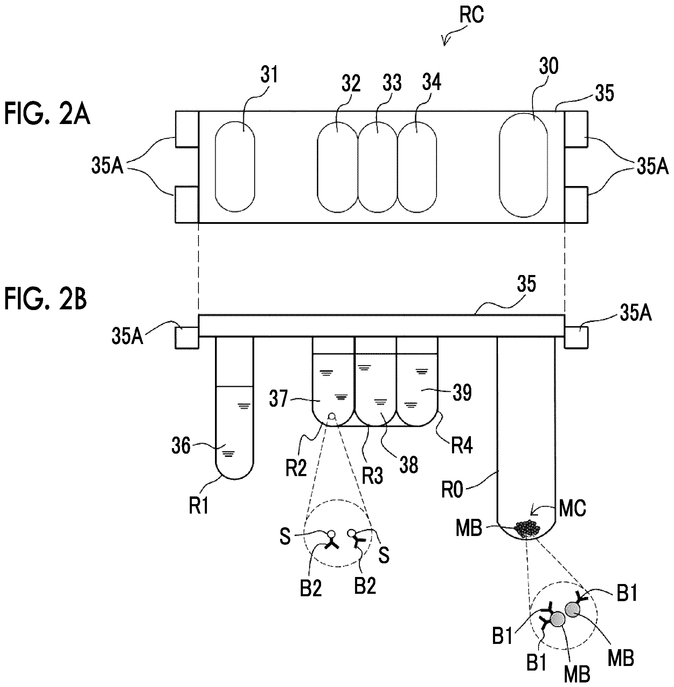

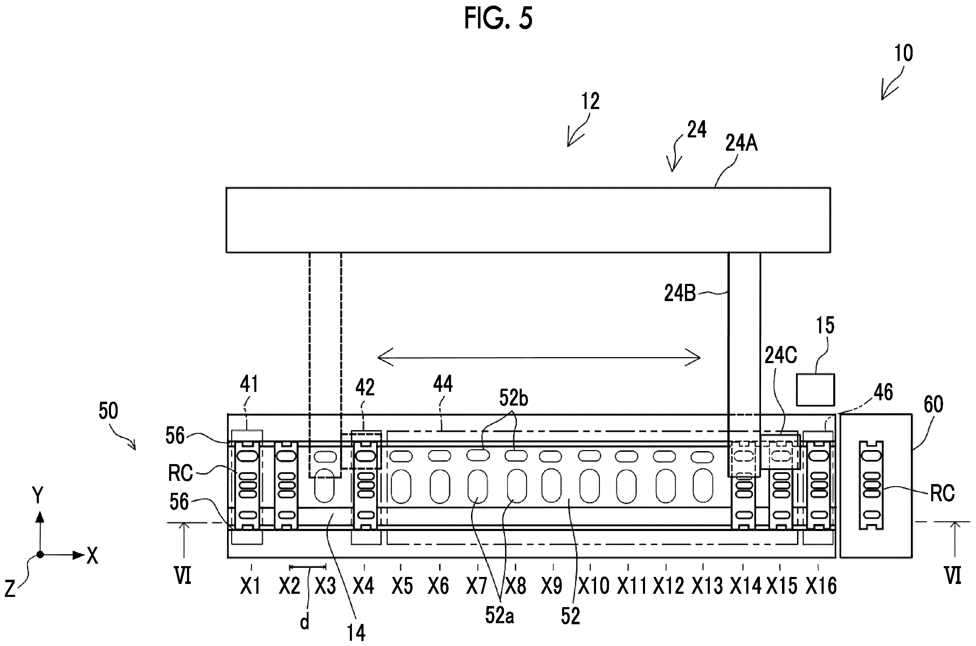

Figs. 2A and 2B are schematic views of an example of the cartridge RC for measurement, in which Fig. 2A is a top view of the cartridge RC and Fig. 2B is a front view of the cartridge RC. The cartridge RC includes a plate-shaped connection portion 35 having five openings 30 to 34, and five tubular cells R0 to R4 each having one end of each of the openings 30 to 34 and extending downward to include the reaction cell R0. Flange portions 35A are provided at both ends of the connection portion 35. The examination apparatus 10 includes a transport mechanism 50 which transports the cartridge RC, and the cartridge RC is held by the transport mechanism 50 and is transported along a transport passage 14 (see Fig. 5) in the transport mechanism 50. The flange portion 35A functions as an engaging portion which engages with the transport mechanism 50. The cartridge RC has a configuration in which the plurality of cells R0 to R4 are integrated by the connection portion 35. Among the plurality of cells R0 to R4, the reaction cell R0 and the cell R1 disposed at both ends are longer than the other cells R2 to R4. The reaction cell R0 is the longest. Before use, the openings 30 to 34 of the cartridge RC are covered with a sealing film (not shown).

The reaction cell R0 contains a plurality of magnetic particles MB modified with a first binding substance B1 for specifically binding to the examination target substance A. The specimen 22 is dispensed into the reaction cell R0, and mixed with various reagents in the reaction cell R0. For example, in a case where the magnetic particles MB have a spherical shape, a diameter thereof is 0.1 to 10 µm, preferably 0.1 to 5 µm, and more preferably approximately 1 to 3 µm.

The cell R1 contains a buffer solution 36. The cell R2 accommodates a labeling reagent 37 containing the label S modified with a second binding substance B2 for specifically binding to the target substance. A first luminescent reagent 38 is contained in the cell R3, and a second luminescent reagent 39 is contained in the cell R4. In the present example, the label S is an enzyme, and the label S emits light in presence of the first luminescent reagent 38 and the second luminescent reagent 39. The labeling reagent 37 in the cell R2, the first luminescent reagent 38 in the cell R3, and the second luminescent reagent 39 in the cell R4 are simply referred to as reagents 37 to 39, in a case where it is not necessary to distinguish the respective liquids.

The first binding substance B1 and the second binding substance B2, which specifically bind to the target substance, are, for example, an antibody against an antigen in a case where the target substance is the antigen, an antigen against an antibody in a case where the target substance is the antibody, and an aptamer against a protein, a low-molecular-weight compound, or the like in a case where the target substance is the protein, the low-molecular-weight compound, or the like. The first binding substance B1 and the second binding substance B2 may be the same or different from each other.

Here, a procedure of the measurement process according to the present example based on the chemiluminescent enzyme immunoassay method will be described with reference to Fig. 3. Fig. 3 schematically shows a reaction in a case where the specimen 22 contains the examination target substance A.

The reaction cell R0 contains the magnetic particles MB modified with the first binding substance B1 in advance, and a buffer solution 36 is dispensed into the reaction cell R0 before the dispensing of the specimen 22. In this state, the specimen 22 is dispensed into the reaction cell R0 (step ST11).

In the reaction cell R0, the magnetic particles MB, the specimen 22, and the buffer solution 36 are mixed with each other, and as a first reaction, a binding reaction in which the examination target substance A in the specimen 22 and the first binding substance B1 are specifically bound to each other occurs (step ST12). In the first reaction, the examination target substance A in the specimen 22 binds to the first binding substance B1, so that the examination target substance A is captured by the magnetic particles MB through the first binding substance B1.

Next, a first cleaning process (B/F separation) for removing unreacted components other than the examination target substance A, which are captured by the magnetic particles MB, is performed (step ST13). A magnet 48 is disposed close to an outside of the reaction cell R0, a reaction liquid after the first reaction is discharged in a state in which the magnetic particles MB are collected on an inner wall surface of the reaction cell R0, and a cleaning liquid 40 is dispensed into the reaction cell R0. In the step ST13 in Fig. 3, a bidirectional arrow in the up-down direction shown at the upper portion of the reaction cell R0 schematically indicates a state in which the cleaning liquid 40 is dispensed into the reaction cell R0 and discharged from the reaction cell R0. In the first cleaning process, the dispensing and the discharging of the cleaning liquid 40 are repeated a plurality of times. The cleaning liquid 40 is also discharged from the reaction cell R0 in a state in which the magnet 48 is disposed close to the outside of the reaction cell R0 and the magnetic particles MB are collected on the inner wall surface of the reaction cell R0.

After the first cleaning process, the labeling reagent 37 is dispensed into the reaction cell R0 (step ST14). The labeling reagent 37 contains a second binding substance B2 to which the label S is imparted, which is a binding substance for specifically binding to the examination target substance A.

In the reaction cell R0, as a second reaction, a binding reaction in which the examination target substance A captured by the magnetic particles MB and the second binding substance B2 are specifically bound to each other (step ST15). As a result, the label S is imparted to the examination target substance A through the second binding substance B2.

Next, a second cleaning process (B/F separation) for removing unreacted components other than the second binding substance B2 bound to the examination target substance A, which are captured by the magnetic particles MB in the labeling reagent 37, is performed (step ST16). The second cleaning process (step ST16) is performed in the same manner as the first cleaning process (step ST13).

Thereafter, the first luminescent reagent 38 and the second luminescent reagent 39 are added to the reaction cell R0 (step ST17). The label S is an enzyme, and causes a chemiluminescence L in the presence of the first luminescent reagent 38 and the second luminescent reagent 39, containing a luminescent substrate. The examination target substance A is detected by detecting the chemiluminescence L (step ST18). The procedure of the measurement process is as described above.

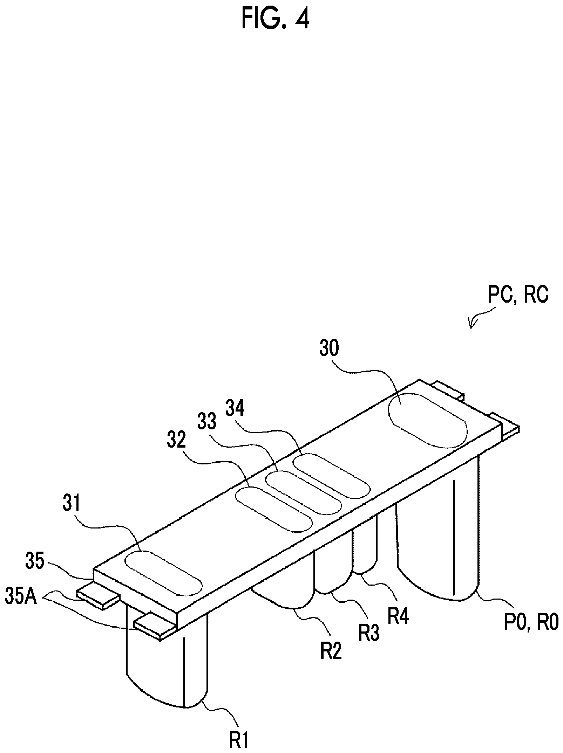

Depending on examination item, the specimen 22 may be pre-treated before the execution of the measurement process in order to improve accuracy of the examination. The pre-treatment is a treatment of heating the specimen 22 in a state of being mixed with a pre-treatment liquid. A cartridge PC for pre-treatment (see Fig. 4) different from the cartridge RC for measurement is used for the pre-treatment. As shown in Fig. 4, the cartridge PC for pre-treatment has a cell P0 for pre-treatment. The cell P0 for pre-treatment accommodates a mixed liquid in which the specimen 22 and a pre-treatment liquid are mixed with each other. In addition, a pre-treatment time which is a heating time for heating the specimen 22 is set to an appropriate time depending on the examination item. In the examination apparatus 10, as will be described later, the pre-treatment time is controlled for each examination item. As information for this, information related to the pre-treatment time such as the pre-treatment time for each examination item and a position on a transport passage 14 (see Fig. 5) for transporting the cartridge PC for pre-treatment in accordance with the pre-treatment time is used. These pieces of information are stored in a memory 17 as setting information.

As shown in Fig. 4, the cartridge PC for pre-treatment in the present example has the same shape as the cartridge RC for measurement. The reaction cell R0 in the cartridge RC for measurement corresponds to the cell P0 for pre-treatment in the cartridge PC for pre-treatment. It is preferable that the cartridge PC for pre-treatment accommodates the pre-treatment liquid in at least one of the cells R1 to R4 before use. In the cartridge PC for pre-treatment, a plurality of cells among the cells R1 to R4 may accommodate different types of pre-treatment liquids. It is sufficient that the cartridge PC for pre-treatment has at least the cell P0 for pre-treatment, which accommodates the mixed liquid of the specimen 22 and the pre-treatment liquid, and has a shape which allows the cartridge PC for pre-treatment to be transported through the transport passage 14 described later; and the cartridge PC for pre-treatment may not have the same shape as the cartridge RC for measurement.

As shown in Fig. 1, the examination apparatus 10 includes, as an example, a specimen transporting unit 11, a dispensing mechanism 12 as a specimen transfer mechanism, a transport mechanism 50, a detecting unit 15, a processor 16, a memory 17, and a touch panel display 18.

The specimen transporting unit 11 has a loading section (not shown) into which the specimen collection container 20 for accommodating the specimen 22 is loaded, and transports the loaded specimen collection container 20 to a position accessible by a nozzle 12A of the dispensing mechanism 12, which will be described later.

The detecting unit 15 executes a detecting process of detecting the examination target substance in the specimen 22. In the present example, the detecting unit 15 is composed of a photodetector such as a photomultiplier tube and a photodiode. The detecting unit 15 optically detects the examination target substance A bound to the label S by receiving the chemiluminescence L. For example, the detecting unit 15 is disposed to face the reaction cell R0 of the cartridge RC for measurement at the detection position, and receives the chemiluminescence L generated from the reaction solution in the reaction cell R0. The detecting unit 15 outputs a received light signal corresponding to an amount of received light to the processor 16.

The processor 16 integrally controls each unit of the examination apparatus 10. An example of the processor 16 is a central processing unit (CPU) which performs various types of control by executing a program. The CPU functions as a control unit which controls each unit by executing a program.

In addition, the processor 16 acquires information on a light amount of the chemiluminescence L in a detecting unit 15, and calculates a density of the examination target substance A based on the information on the light amount.

The memory 17 is an example of a memory connected to or built into the CPU as the processor 16. For example, the memory 17 stores a control program. In addition to the control program, the memory 17 stores setting information which is preset in order for the processor 16 to perform the various types of control.

In addition, the memory 17 stores information indicating a correspondence relationship between the light amount of the chemiluminescence L detected by the detecting unit 15 and the amount of the examination target substance A. The correspondence relationship is stored, for example, as a calibration curve represented by a function. The correspondence relationship may be a format of a table. The processor 16 calculates the amount of the examination target substance A from, for example, the light amount of the chemiluminescence L acquired from the detecting unit 15, and the calibration curve stored in the memory 17.

The touch panel display 18 receives, from a user, an operation instruction such as an instruction to start the examination. In addition, the touch panel display 18 displays information such as an examination result.

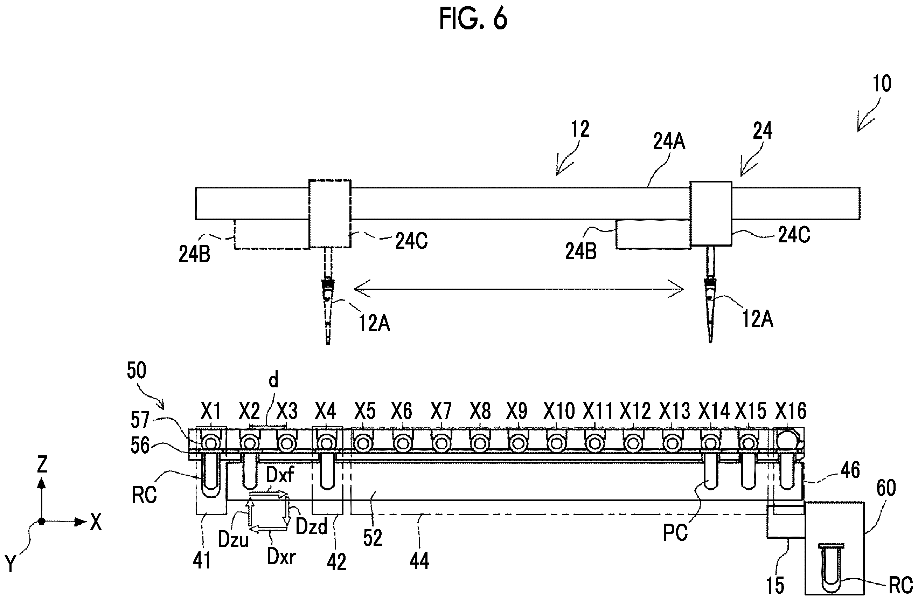

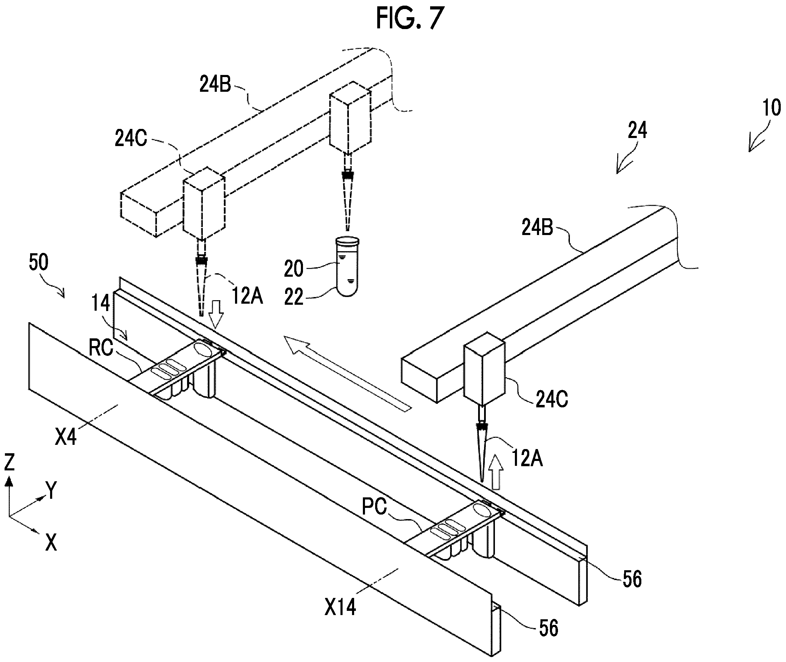

Figs. 5 to 7 are schematic views showing a configuration of the transport mechanism 50 and the dispensing mechanism 12, in which Fig. 5 is a plan view, Fig. 6 is a cross-sectional view taken along a line VI-VI in Fig. 5, and Fig. 7 is a perspective view. In the drawings, a transport direction of the transport passage 14 is referred to as an X direction, a direction orthogonal to the X direction in a horizontal plane is referred to as a Y direction, and a vertical direction perpendicular to an X-Y plane is referred to as a Z direction. The directions indicated by arrows X, Y, and Z in the following drawings match each other.

The transport mechanism 50 has a linear transport passage 14 that transports both cartridges of the cartridge RC for measurement and the cartridge PC for pre-treatment. A start end part of the transport passage 14 is a cartridge set position 41 at which each cartridge of the cartridge RC for measurement and the cartridge PC for pre-treatment is set. Each cartridge is transported to a terminal end on the downstream side of the transport passage 14 with the cartridge set position 41 as an upstream end in the transport direction. A specimen dispensing position 42 at which the specimen 22 is dispensed to each cartridge is provided on the downstream side of the cartridge set position 41 along the transport passage 14. Furthermore, a processing section 44 and a detection position 46 are provided on the downstream side in this order. The detection position 46 is positioned at a terminal end part of the transport passage 14. The detecting unit 15 is disposed to be able to detect the light L from the reaction cell R0 of the cartridge RC for measurement at the detection position 46. At the terminal end of the transport passage 14, a cartridge discarding unit 60 that the cartridge RC for measurement, in which the measurement has been completed, and the cartridge PC for pre-treatment used for the pre-treatment are discarded is provided below in a vertical direction orthogonal to the transport direction. The cartridge RC for measurement is transported along the transport passage 14, and is sequentially transported to the specimen dispensing position 42, the processing section 44, and the detection position 46. At the specimen dispensing position 42, specimen dispensing (step ST11) in the measurement procedure shown in Fig. 3 is performed. At the processing section 44, each piece of processes from first reaction (step ST12) to addition of a luminescent reagent (step ST17) in the measurement procedure shown in Fig. 3 is sequentially performed. At the detection position 46, detecting process (step ST18) of the measurement procedure shown in Fig. 3 is performed.

On the transport passage 14, the processing section 44 includes a reagent dispensing mechanism (not shown) that includes a nozzle for suctioning the reagents 37 to 39 and dispensing each of the reagents into the reaction cell R0, and a cleaning nozzle for discharging the cleaning liquid 40 to the reaction cell R0 and suctioning and discharging the cleaning liquid 40 after the cleaning process. The reagent dispensing mechanism includes a moving mechanism and a suction-discharge mechanism, similarly to the dispensing mechanism 12 described later.

The transport mechanism 50 includes a heat block 52 functioning as a heating unit, a pair of guide parts 56 which are long in the transport direction for guiding the transport of the cartridge RC for measurement and the cartridge PC for pre-treatment, and a plurality of rotating bodies 57 (see Fig. 6; not shown in Fig. 5 and Fig. 7) which are arranged on each guide part 56 at regular intervals along the guide parts 56. In the present example, the transport passage 14 is a space defined by the guide part 56 on the side and the heat block 52 below. The cartridge RC for measurement and the cartridge PC for pre-treatment are held by the transport mechanism 50 by engaging with the guide 56, and are transported along the transport passage 14. The transport mechanism 50 can transport the cartridge RC for measurement and the cartridge PC for pre-treatment while heating the cells R2 to R4 and the reaction cell R0.

As shown in Fig. 6, the rotating bodies 57 are arranged at regular intervals of a distance d. As shown in Figs. 5 and 6, the X direction positions of the rotating bodies 57 arranged at regular intervals in the transport direction are represented by X1, X2, X3, ..., and X16 from the start end side of the transport passage 14. The cartridge set position 41 is set to the position X1 at a start end of the transport passage 14, and the specimen dispensing position 42 is set to the position X4. In addition, the processing section 44 is provided within a range from the position X5 to the position X15, and the detection position 46 is provided at the position X16.

Each of the guide parts 56 of the pair of guide parts 56 supports the flange portion 35A of the cartridge RC for measurement. As a result, the cartridge RC for measurement is suspended between the pair of guide parts 56. At each of the positions X1 to X16 where the rotating bodies 57 are provided, the flange portion 35A is sandwiched between the guide part 56 and the rotating body 57. In a case where the cartridge RC for measurement is moved, the rotating body 57 rotates with movement of the cartridge RC for measurement to assist the movement of the measurement cartridge RC.

In addition, a plurality of sets of recess portions, in which a first recess portion 52a (see Fig. 5) and a second recess portion 52b (see Fig. 5) are set as one set, are provided in the heat block 52 along the transport direction. A disposition interval between the first recess portion 52a and the second recess portion 52b is the same distance d as the disposition interval of the rotating bodies 57 described above, and is the same as the disposition interval between the respective positions X1 to X16. The heat block 52 is one of constituent elements of the transport mechanism 50, and the transport mechanism 50 includes a driving unit (not shown) which is an actuator for moving the heat block 52.

The heat block 52 heats the cells R2 to R4 and the reaction cell R0. In the transport passage 14, the cartridge RC for measurement and the cartridge PC for pre-treatment are set in a horizontal posture in which an arrangement direction of the cells R2 to R4 and the reaction cell R0 is orthogonal to the transport direction. One set of the first recess portion 52a and the second recess portion 52b is formed along the arrangement direction of the cells R2 to R4 and the reaction cell R0. In the cartridge RC for measurement and the cartridge PC for pre-treatment, the cells R2 to R4 and the reaction cell R0 are inserted into the first recess portion 52a and the second recess portion 52b, and the cells R2 to R4 and the reaction cell R0 are heated by the insertion into the first recess portion 52a and the second recess portion 52b. The heat block 52 is connected to a heater (not shown) and is temperature-controlled by a temperature controller which controls the heater. A temperature of the heat block 52 is set to approximately 36°C to 38°C, preferably 37°C, from the viewpoint of promoting an immunoreaction.

The cartridge RC for measurement, in a state in which the cells R2 to R4 are inserted into the first recess portion 52a and the reaction cell R0 is inserted into the second recess portion 52b, is transported by the transport passage 14 by moving the heat block 52 in the transport direction (that is, the X direction). The cartridge RC for measurement is supported by the guide part 56, but can be moved in the transport direction by the rotation of the rotating body 57. Therefore, in a case where the heat block 52 into which the cells R2 to R4 and the like are inserted, which is a part of the cartridge RC for measurement, is moved in the transport direction, the cartridge RC for measurement in a state of being supported by the guide part 56 is moved in the transport direction. Similarly, the cartridge PC for pre-treatment, in a state in which the cells R2 to R4 are inserted into the first recess portion 52a, is transported by moving the heat block 52 in the transport direction (that is, the X direction), and thus the transport passage 14 is transported in a state in which the cell P0 for pre-treatment is inserted into the second recess portion 52b. In practice, the heat block 52 holds a plurality of the cartridges RC for measurement and the cartridges PC for pre-treatment. In this state, the heat block 52 is moved, and thus the plurality of cartridges RC for measurement and the cartridges PC for pre-treatment are collectively transported in the transport direction.

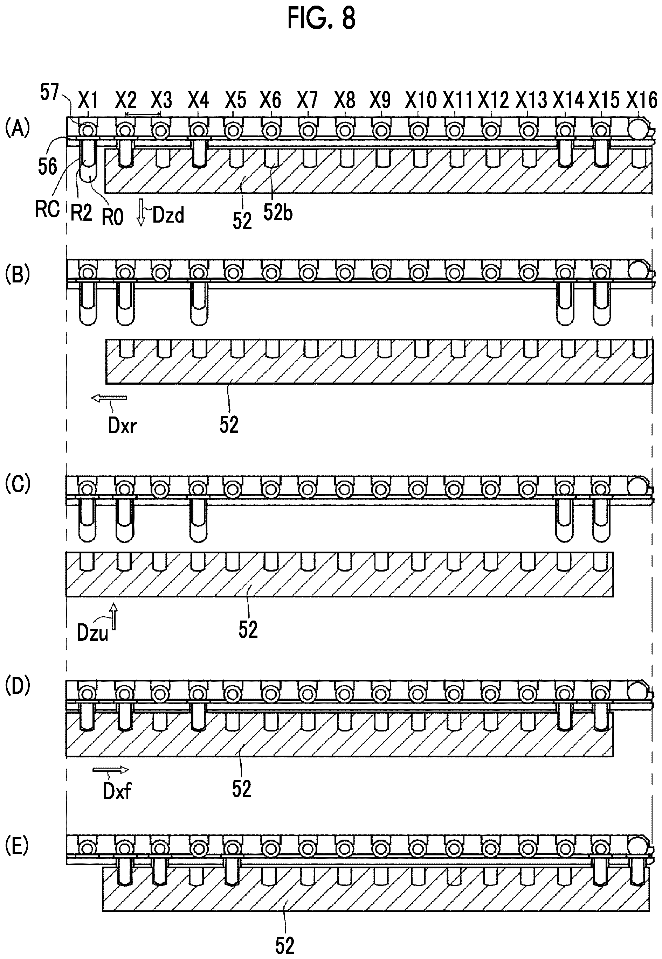

Fig. 8 schematically shows a transporting method using the heat block 52. The driving unit of the transport mechanism 50 can move the heat block 52 along four directions of arrows Dzd, Dxr, Dzu, and Dxf shown in Fig. 8 (see also Fig. 6). The movement along the arrow Dzd is movement downward in the vertical direction, and the movement along the arrow Dxr is movement upstream in the transport direction (that is, the X direction) in the horizontal direction. The movement along the arrow Dzu is movement upward in the vertical direction, and the movement along the arrow Dxf is movement downstream in the transport direction (that is, the X direction) in the horizontal direction. In a case where the operation of moving the cartridge RC for measurement in the four directions of the arrows Dzd, Dxr, Dzu, and Dxf in this order is set as a single period operation of the heat block 52, the cartridge RC for measurement and the cartridge PC for pre-treatment are moved by the distance d in the transport direction with the single period operation of the heat block 52. A moving distance in the ±X direction along the arrows Dxr and Dxf is the same distance d as the arrangement period of the rotating bodies 57. Therefore, for example, the cartridge RC for measurement or the cartridge PC for pre-treatment at the position X1 is moved to the position X2 adjacent to the downstream side of the position X1 with the single period operation of the heat block 52.

The transporting method using the heat block 52 will be described in more detail with reference to Fig. 8. Fig. 8 shows a cross section along the transport direction, which is a cross section in which the cell R2 of the cartridge RC inserted into the first recess portion 52a and the first recess portion 52b is visible. First, (A) of Fig. 8 shows a state in which the cells R2 to R4 and the reaction cell R0 of the cartridge RC are inserted into the first recess portion 52a and the second recess portion 52b of the heat block 52 shown in Fig. 6, respectively. In this way, the heat block 52 is moved downward along the arrow Dzd from the state in which the cartridge RC is inserted into the heat block 52. (B) of Fig. 8 shows a state in which the heat block 52 is moved downward. From the state of (B) of Fig. 8, the heat block 52 is moved by the distance d to the upstream side in the transport direction along the arrow Dxr. (C) of Fig. 8 shows a state in which the heat block 52 is moved by the distance d to the upstream side in the transport direction. The heat block 52 is moved upward along the arrow Dzu from the state of (C) in Fig. 8. (D) of Fig. 8 shows a state in which the heat block 52 is moved upward. In (A) of Fig. 8, the cartridge RC for measurement at the position X1, which is not inserted into the heat block 52, is inserted into the heat block 52 in (D) of Fig. 8. From the state of (D) in Fig. 8, the heat block 52 is moved by the distance d in the transport direction along the arrow Dxf. As a result, as shown in (E) of Fig. 8, the cartridge RC at the position X1 in (A) of Fig. 8 is moved to the position X2.

In this way, the cartridge RC is transported from the position X1 corresponding to the cartridge set position 41 to the position X16 corresponding to the detection position 46, by repeating the movement of the heat block 52 by the single period operation in the four directions of the arrows Dzd, Dxr, Dzu, and Dxf in the transport direction. After the detecting process is performed at the position X16 corresponding to the detection position 46, the cartridge RC is pushed out from the transport passage 14 with the movement of the heat block 52, falls downward from the terminal end of the transport passage 14, and is accommodated in the cartridge discarding unit 60 (see Fig. 6).

The cartridge PC for pre-treatment is sequentially transported on the transport passage 14 from the cartridge set position 41 to the specimen dispensing position 42, the processing section 44, and the detection position 46, similarly to the cartridge RC for measurement. The cartridge PC for pre-treatment is transported in a state in which the cells R2 to R4 are inserted into the first recess portion 52a, the cell P0 for pre-treatment is inserted into the second recess portion 52b, and the cells are heated by the heat block 52. That is, the transport passage 14 transports the cartridge PC for pre-treatment while performing the pre-treatment on the specimen 22. The processor 16 reads out the pre-treatment time according to the examination item of the specimen 22 from the memory 17, and transports the cartridge PC for pre-treatment to a position corresponding to the pre-treatment time.

The dispensing mechanism 12 is a mechanism which dispenses a liquid, and includes a nozzle 12A which suctions and discharges the specimen 22, a nozzle moving mechanism 24 (see Fig. 5), and a suction-discharge mechanism (not shown). The nozzle 12A is provided with a replaceable tip at a distal end thereof. The nozzle moving mechanism 24 includes an X direction movable part 24A, a Y direction movable part 24B, and a Z direction movable part 24C, and is a mechanism that three-dimensionally moves the nozzle 12A in a vertical direction (Z direction) and a horizontal direction (X-Y direction). The suction-discharge mechanism is a mechanism that causes the nozzle 12A to perform a suction operation of suctioning the liquid and a discharge operation of discharging the liquid from the nozzle 12A. Each of the movable parts 24A to 24C of the nozzle moving mechanism 24 includes an actuator such as a motor, which generates a driving force, and a driving force transmission mechanism consisting of a gear, a drive belt, and the like. The suction-discharge mechanism is composed of a pump or the like, which generates a driving force for the suction and discharge.

The dispensing mechanism 12 performs suction and discharge of the specimen 22 by the nozzle 12A. Specifically, as shown in Fig. 7, the dispensing mechanism 12 suctions the pre-treated specimen 22 from the cell P0 for pre-treatment of the cartridge PC for pre-treatment at any position (for example, the position X14) on the transport passage 14. The dispensing mechanism 12 moves the nozzle 12A to the specimen dispensing position 42 at the position X4, and discharges the pre-treated specimen 22 to the reaction cell R0 of the cartridge RC for measurement, positioned at the specimen dispensing position 42. As described above, in the present embodiment, the dispensing mechanism 12 constitutes a specimen transfer mechanism that transfers the pre-treated specimen 22 which has been subjected to the pre-treatment, from the cartridge PC for pre-treatment transported to any position on the transport passage 14 to the cartridge RC for measurement disposed at the specimen dispensing position 42. The any position in the transport direction in which the cartridge PC for pre-treatment is transported is a position determined according to the examination item of the specimen 22; and in the present example, the any position is any position of X5 to X15 between the position X4 corresponding to the specimen dispensing position 42 and the position X16 corresponding to the detection position 46.

In a case where it is not necessary to perform the pre-treatment on the specimen 22, the dispensing mechanism 12 is also used for dispensing the specimen 22 which has not been subjected to the pre-treatment into the reaction cell R0 of the cartridge RC for measurement. That is, in a case where the specimen 22 does not need the pre-treatment, the dispensing mechanism 12 suctions the specimen 22 from the specimen collection container 20, and dispenses the specimen 22 into the reaction cell R0 of the cartridge RC for measurement disposed at the specimen dispensing position X4 (see Fig. 7). As described above, the dispensing mechanism 12 may also serve as a specimen transfer mechanism and a specimen dispensing mechanism.

The action of the above-described configuration will be described. Examination of a case in which the specimen 22 on which the pre-treatment has been performed is subjected to the measurement process will be described.

A specimen collection container 20 which accommodates the specimen 22 to be examined is loaded into the examination apparatus 10. In the examination apparatus 10, a plurality of the cartridges PC for pre-treatment and a plurality of the cartridges RC for measurement are set in a loading section (not shown) in advance.

In a case where examination order for the specimen 22 requiring the pre-treatment for the specimen 22 is received, the processor 16 controls a cartridge set mechanism (not shown) to set the cartridge PC for pre-treatment at the position X1 corresponding to the cartridge set position 41 of the transport passage 14. The transport mechanism 50 intermittently transports the cartridge on the transport passage 14 in the transport direction by moving the heat block 52 along the arrows DzdDzd, Dxr, Dzu, and Dxf shown in (A) to (D) of Fig. 8 at regular time intervals by the distance d.

Next, the processor 16 controls the reagent dispensing mechanism (not shown) to dispense the pre-treatment liquid to be mixed with the specimen 22 to the cell P0 for pre-treatment of the cartridge PC for pre-treatment at the position X2. The pre-treatment liquid is accommodated in, for example, the cell R1 in the cartridge PC for pre-treatment, and the pre-treatment liquid in the cell R1 is dispensed into the cell P0 for pre-treatment by the reagent dispensing mechanism including a nozzle for dispensing the pre-treatment liquid.

Thereafter, the processor 16 controls the dispensing mechanism 12 to suction the specimen 22 accommodated in the specimen collection container 20 at the position X4 corresponding to the specimen dispensing position 42 by the nozzle 12A, and to dispense the specimen 22 into the cell P0 for pre-treatment. In this case, the suction and discharge operation is repeated in the cell P0 for pre-treatment by the nozzle 12A, and thus the specimen 22 and the pre-treatment liquid are mixed with each other.

Thereafter, the cartridge PC for pre-treatment is transported to the downstream side along the transport passage 14. In this case, the cell P0 for pre-treatment is transported in a state in which the heat block 52 is inserted into the second recess portion 52b. Therefore, while the cartridge PC for pre-treatment is transported, the specimen 22 mixed with the pre-treatment reagent in the cell P0 for pre-treatment is heated by the heat block 52, and is subjected to the pre-treatment.

The processor 16 transports the cartridge PC for pre-treatment to any position according to the pre-treatment time determined for each examination item by the transport mechanism 50. As an example, the cartridge PC for pre-treatment is transported to the position X14 shown in Fig. 6.

The processor 16 controls the dispensing mechanism 12 such that the nozzle 12A suctions the pre-treated specimen 22 from the cell P0 for pre-treatment of the cartridge PC for pre-treatment at the position X14. Next, the processor 16 moves the nozzle 12A from the position X14 to the position X4 corresponding to the specimen dispensing position 42, and dispenses the pre-treated specimen 22 to the reaction cell R0 of the cartridge RC for measurement at the specimen dispensing position 42.

The processor 16 controls a cartridge set mechanism (not shown) to set the cartridge RC for measurement at the position X1 corresponding to the cartridge set position 41 of the transport passage 14 during the transporting of the cartridge PC for pre-treatment. In the present example, in a case where the cartridge PC for pre-treatment is transported to the position where the pre-treatment is completed (here, the position X14), the cartridge RC for measurement is set at the position X1 corresponding to the cartridge set position 41, at a timing at which the cartridge RC for measurement is transported to the position X4 corresponding to the specimen dispensing position 42.

The cartridge PC for pre-treatment, having the cell P0 for pre-treatment from which the pre-treated specimen 22 is suctioned, is transported to the downstream side as it is, and is pushed out from the transport passage 14 at the terminal end of the transport passage 14, falls down, and is accommodated in the cartridge discarding unit 60 (see Fig. 6).

On the other hand, the cartridge RC for measurement, having the reaction cell R0 in which the pre-treated specimen 22 is dispensed, is transported on the transport passage 14, and the processes are performed in the processing section 44 and the detecting unit 15, arranged along the transport passage 14. Specifically, in the processing section 44, each piece of processes from the first reaction (step ST12) to the addition of the luminescent reagent (step ST17) in the measurement procedure shown in Fig. 3 is sequentially performed, and the detecting process (step ST18) in the measurement procedure shown in Fig. 3 is performed at the detecting unit 15.

Similarly to the cartridge PC for pre-treatment, the cartridge RC for measurement is pushed out from transport passage 14 at the terminal end of the transport passage 14, falls down, and is accommodated in the cartridge discarding unit 60.

As described above, the examination apparatus 10 which is an example of the embodiment includes the linear transport passage 14 that transports both cartridges of the cartridge PC for pre-treatment and the cartridge RC for measurement; and a specimen transfer mechanism (in the present example, the dispensing mechanism 12) that transfers the pre-treated specimen 22 in which the pre-treatment is completed, from the cartridge PC for pre-treatment transported at any position between, in the transport passage 14, the position X4 corresponding to the specimen dispensing position 42, where the specimen 22 is dispensed into the reaction cell R0 of the cartridge RC for measurement, and the position X16 corresponding to the detection position 46, where the examination target substance A in the specimen 22 is optically detected in the downstream of the transport passage 14 in the transport direction with respect to the specimen dispensing position 42, to the cartridge RC for measurement disposed at the position X4 corresponding to the specimen dispensing position 42. With the present configuration, the pre-treated specimen 22 can be transferred from the cartridge PC for pre-treatment transported to the any position of the transport passage 14 to the cartridge for measurement positioned at the specimen dispensing position X4. Therefore, the pre-treatment can be performed for each examination item (examination target substance) for an appropriate pre-treatment time.

In the examination apparatus 10, since the pre-treatment is performed in the transport passage 14 in which the measurement process is performed, it is not necessary to newly provide a processing section for the pre-treatment separately from the transport passage for the measurement process. Therefore, the examination apparatus 10 can perform the pre-treatment for each examination item in an appropriate heating time while suppressing an increase in size.

In the present example, the examination apparatus includes, as the specimen transfer mechanism, the dispensing mechanism 12 that performs suction and discharge of a liquid by the nozzle 12A, and the dispensing mechanism 12 transfers the pre-treated specimen 22 by suctioning the pre-treated specimen 22 from the cell P0 for pre-treatment of the cartridge PC for pre-treatment at the any position on the transport passage 14, and dispensing the pre-treated specimen 22 into the reaction cell R0 of the cartridge RC for measurement at the specimen dispensing position X4. The dispensing mechanism 12 includes the X direction movable part 24A that moves the nozzle 12A in the X direction which is the transport direction, but the examination apparatus can be configured to be small as compared with a case where the nozzle 12A is moved for each cartridge in the transport direction (refers to a second embodiment described later).

In the present example, the dispensing mechanism 12 is also used for dispensing the specimen 22 which has not been subjected to the pre-treatment into the reaction cell R0, in addition to transferring the pre-treated specimen 22 from the cell P0 for pre-treatment to the reaction cell R0. However, a dispensing mechanism as a specimen transfer mechanism which transfers the pre-treated specimen 22 from the cell P0 for pre-treatment to the reaction cell R0, and a specimen dispensing mechanism which dispenses the un-treated specimen 22 to the reaction cell R0 may be separately provided. In this case, the specimen dispensing mechanism is used not only for dispensing the specimen 22, which does not need the pre-treatment, to the reaction cell R0, but also for dispensing the specimen 22, which needs the pre-treatment, to the cell P0 for pre-treatment.

However, as in the present example, in a case where the dispensing mechanism 12 is also used for dispensing the specimen 22 which has not been subjected to the pre-treatment, into the reaction cell R0, in addition to transferring the specimen 22 which has been subjected to the pre-treatment, from the cell P0 for pre-treatment to the reaction cell R0, the examination apparatus can be configured to be smaller than in a case where two dispensing mechanisms are provided.

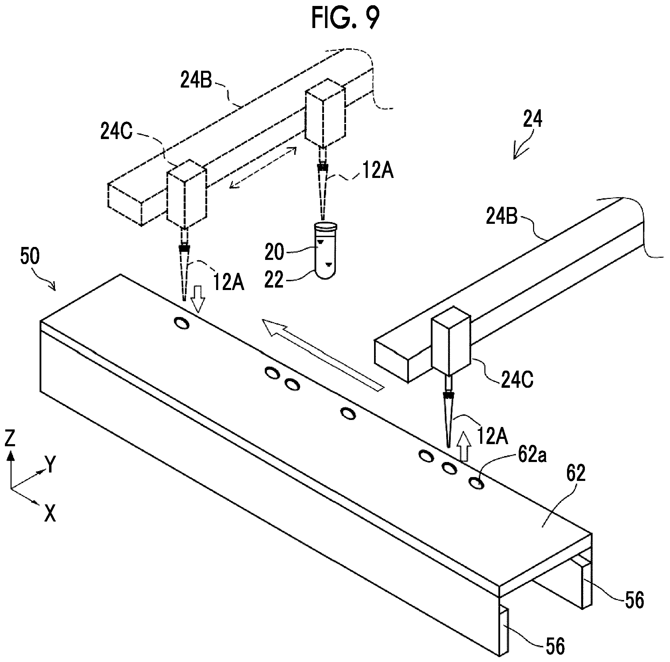

In the examination apparatus 10 of the present example, a plurality of the cartridges RC for measurement and a plurality of the cartridges PC for pre-treatment, which are used for different specimens 22, are sequentially transported on the transport passage 14. In such an examination apparatus 10, it is preferable to include a cover member 62 that can cover the cartridges RC for measurement and the cartridges PC for pre-treatment above the transport passage 14, as in a modification example shown in Fig. 9. The cover member 62 is provided to prevent the specimen 22 transferred by the dispensing mechanism 12 from being mixed into the cartridge RC for measurement and the cartridge PC for pre-treatment, which are used for another specimen. The cover member 62 shown in Fig. 9 has a shape that covers the upper side of the transport passage 14 and is disposed on the transport passage 14. The cover member 62 is provided with a through-hole 62a for inserting the nozzle 12A for dispensing the specimen into the cell P0 for pre-treatment, suctioning the specimen, dispensing the specimen into the reaction cell R0, and the like. Although not shown, the cover member 62 is also provided with a through-hole for inserting a nozzle for each reagent, that suctions the reagents 36 to 39 from the cells R1 to R4. Although the cover member 62 is shown to be composed of one member in Fig. 9, the cover member 62 may be composed of a plurality of members. In addition, the cover member 62 is not limited to the shape and size of Fig. 9 as long as it can prevent liquid dripping from the nozzle 12A in a case of moving the nozzle 12A from being mixed into the cartridge RC for measurement and the cartridge PC for pre-treatment, which are used for another specimen.

In a case where a plurality of the cartridges RC for measurement and a plurality of the cartridges PC for pre-treatment, which are used for different specimens, are sequentially transported on the transport passage 14 as in the examination apparatus 10 of the embodiment described above, there is a concern that dripping and liquid splashing may occur in a case where the nozzle 12A is moved to the specimen dispensing position X4 in a state in which the pre-treated specimen 22 is suctioned by the dispensing mechanism 12. As described above, in a case where the cover member 62 that can cover the cartridges RC for measurement and the cartridges PC for pre-treatment above the transport passage 14 and prevents the specimen 22 transferred by the dispensing mechanism 12 from being mixed with the cartridge RC for measurement and the cartridge PC for pre-treatment, which are used for another specimen, is provided, the contamination can be suppressed. In addition, by providing the cover member 62, it is possible to suppress not only dripping of liquid during the movement of the nozzle 12A but also mixing of dust and the like.

### "Examination apparatus according to second embodiment"

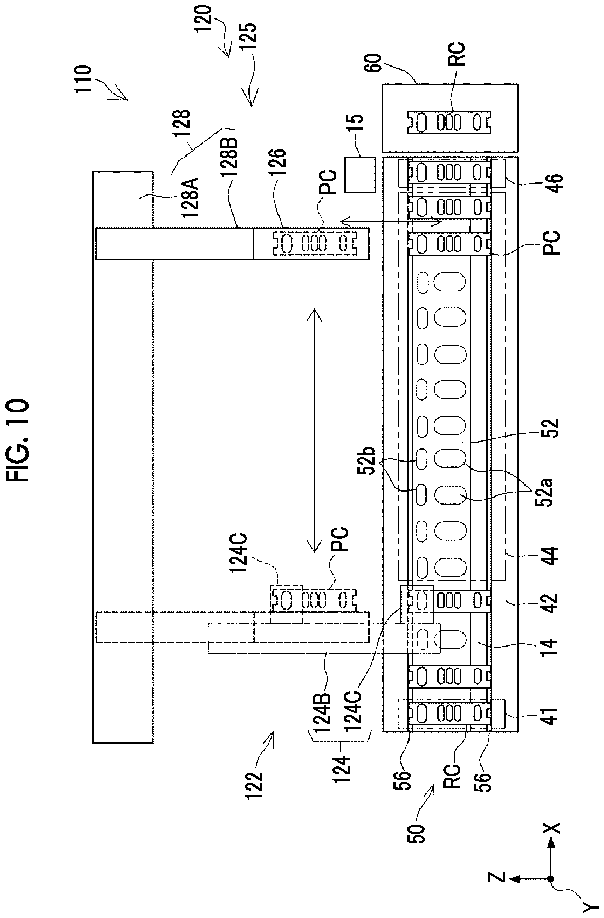

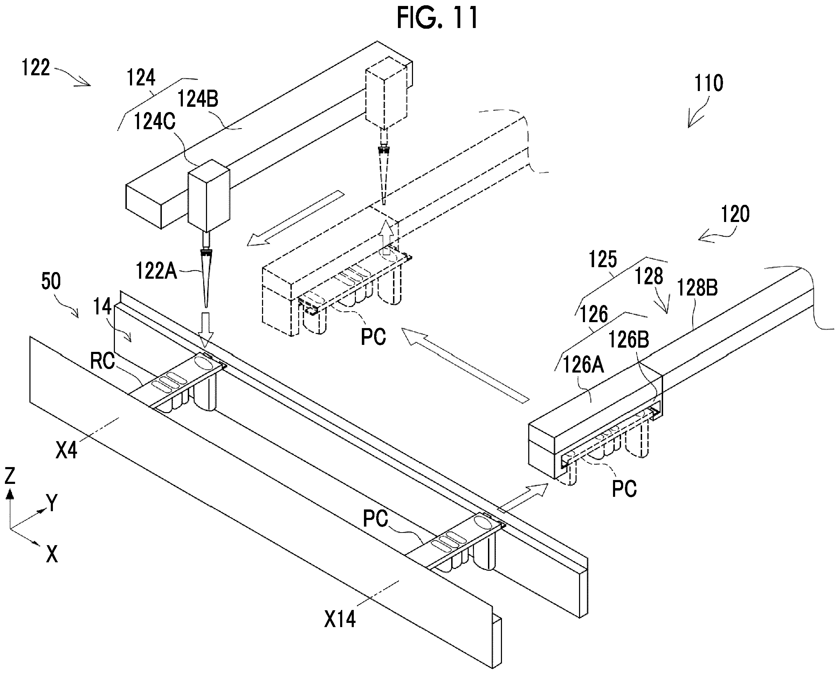

Figs. 10 and 11 are views showing a schematic configuration of a transport passage 14 and a specimen transfer mechanism 120 in an examination apparatus 110 according to a second embodiment, in which Fig. 10 is a plan view and Fig. 11 is a perspective view. In Figs. 10 and 11, the same constituent elements as those of the examination apparatus 10 are denoted by the same reference numerals, and detailed description thereof will be omitted. Hereinafter, the examination apparatus 110 according to the second embodiment will be described mainly with differences from the examination apparatus 10 according to the first embodiment.

In the examination apparatus 10 according to the first embodiment, the dispensing mechanism 12 constitutes the specimen transfer mechanism, but in the examination apparatus 110 according to the second embodiment, the specimen transfer mechanism 120 includes a dispensing mechanism 122 and a pre-treatment cartridge moving mechanism 125.

The pre-treatment cartridge moving mechanism 125 includes, for example, a pre-treatment cartridge gripping portion 126 and a gripping part moving mechanism 128.

The pre-treatment cartridge gripping portion 126 grips the cartridge PC for pre-treatment. The pre-treatment cartridge gripping portion 126 includes a support part 126A and a displacement part 126B that grips the flange portion 35A of the cartridge PC for pre-treatment and changes the position of the cartridge PC for pre-treatment with respect to the support part 126A. The displacement part 126B can be switched between a state of supporting the cartridge PC for pre-treatment below the support part 126A and a state of supporting the cartridge PC for pre-treatment on a side of the support part 126A. In a case where the pre-treatment cartridge gripping portion 126 grips and moves the cartridge PC for pre-treatment, the pre-treatment cartridge gripping portion 126 brings the cartridge PC for pre-treatment into a state of being supported below the support part 126A. In a case of suctioning the specimen 22, the cartridge PC for pre-treatment is supported on the side of the support part 126A. In Fig. 11, the pre-treatment cartridge gripping portion 126 shown in the vicinity of the position X14 of the transport passage 14 shows a state in which the cartridge PC for pre-treatment is supported below the support part 126A; and the pre-treatment cartridge gripping portion 126 in the vicinity of the position X4, shown by a broken line, shows a state in which the cartridge PC for pre-treatment is supported on the side of the support part 126A.

The gripping part moving mechanism 128 includes an X direction movable part 128A that moves the pre-treatment cartridge gripping portion 126 in the transport direction, a Y direction movable part 128B that moves the pre-treatment cartridge gripping portion 126 in a horizontal plane in a Y direction orthogonal to the transport direction, and a Z direction movable part (not shown) that moves the pre-treatment cartridge gripping portion 126 in a vertical direction (Z direction). Each of the X direction movable part 128A, the Y direction movable part 128B, and the Z direction movable part includes an actuator such as a motor, which generates a driving force, and a driving force transmission mechanism consisting of a gear, a drive belt, and the like.

The pre-treatment cartridge moving mechanism 125 detaches the cartridge PC for pre-treatment from the transport passage 14, and moves the cartridge PC for pre-treatment to the upstream side in the transport direction of the transport passage 14. More specifically, as shown in Fig. 11, the pre-treatment cartridge moving mechanism 125 detaches, from the transport passage 14, the cartridge PC for pre-treatment at any position (in the figure, the position X14) on the transport passage 14, and moves the cartridge PC for pre-treatment to the vicinity of the specimen dispensing position X4.

The dispensing mechanism 122 is a mechanism which dispenses a liquid, and includes a nozzle 122A which suctions and discharges the specimen 22, a nozzle moving mechanism 124, and a suction-discharge mechanism (not shown). The nozzle 122A is provided with a replaceable tip at a distal end thereof. The nozzle moving mechanism 124 includes a Y direction moving mechanism 124B and a Z direction moving mechanism 124C, and is a mechanism that moves the nozzle 122A in a vertical direction (Z direction) and a horizontal direction (Y direction). The nozzle moving mechanism 124 may further include an X-direction moving mechanism. The suction-discharge mechanism is a mechanism that causes the nozzle 122A to perform a suction operation of suctioning the liquid and a discharge operation of discharging the liquid from the nozzle 122A. The moving mechanism 124 includes an actuator such as a motor, which generates a driving force, and a driving force transmission mechanism consisting of a gear, a drive belt, and the like. The suction-discharge mechanism is composed of a pump or the like, which generates a driving force for the suction and discharge.

The dispensing mechanism 122 suctions, by the nozzle 122A, the pre-treated specimen 22 from the cell P0 for pre-treatment of the cartridge PC for pre-treatment moved to the vicinity of the specimen dispensing position X4 by the pre-treatment cartridge moving mechanism 125, and discharges the pre-treated specimen 22 to the reaction cell R0 of the cartridge RC for measurement disposed at the specimen dispensing position X4.

In this way, the specimen transfer mechanism 120 moves the cartridge PC for pre-treatment by the pre-treatment cartridge moving mechanism 125, and then transfers the pre-treated specimen 22 from the cell P0 for pre-treatment to the reaction cell R0 by the dispensing mechanism 122.

Similarly to the dispensing mechanism 12, in a case where it is not necessary to perform the pre-treatment on the specimen 22, the dispensing mechanism 122 is also used for dispensing the specimen 22 which has not been subjected to the pre-treatment into the reaction cell R0 of the cartridge RC for measurement. That is, in a case where the specimen 22 does not need the pre-treatment, the dispensing mechanism 122 suctions the specimen 22 from the specimen collection container 20, and dispenses the specimen 22 into the reaction cell R0 of the cartridge RC for measurement disposed at the specimen dispensing position X4. As described above, the dispensing mechanism 122 may also serve as a specimen transfer mechanism and a specimen dispensing mechanism.

The action of the present examination apparatus 110 having the above-described configuration is the same as that of the examination apparatus 10, except that the pre-treated specimen 22 is transferred by the pre-treatment cartridge moving mechanism 125 and the dispensing mechanism 122.

As described above, the examination apparatus 110 according to the second embodiment includes, as the specimen transfer mechanism 120, the dispensing mechanism 122 that performs suction and discharge of a liquid by the nozzle 122A, and the pre-treatment cartridge moving mechanism 125 that detaches the cartridge PC for pre-treatment from the transport passage 14 and moves the cartridge PC for pre-treatment to the upstream side in the transport direction of the transport passage 14. After moving the cartridge PC for pre-treatment by the pre-treatment cartridge moving mechanism 125, the dispensing mechanism 122 transfers the pre-treated specimen 22 from the cell P0 for pre-treatment to the reaction cell R0. According to the present configuration, since the cartridge PC for pre-treatment can be moved to the vicinity of the cartridge RC for measurement, the distance over which the pre-treated specimen 22 is held and moved by the nozzle 122A is shortened, and thus the contamination risk due to dripping can be suppressed.

In addition, in the present example, in a case where the pre-treatment cartridge gripping portion 126 grips and moves the cartridge PC for pre-treatment, the pre-treatment cartridge gripping portion 126 brings the cartridge PC for pre-treatment into a state of being supported below the support part 126A. In a case of suctioning the specimen 22, the cartridge PC for pre-treatment is supported on the side of the support part 126A. Accordingly, in a case where the cartridge PC for pre-treatment is moved, the support part 126A can cover the upper surface of the cartridge PC for pre-treatment. Therefore, it is possible to suppress the mixing of dust and the like in the specimen 22.

In addition, even in the present example, since the dispensing mechanism 122 is also used for dispensing the specimen 22 which has not been subjected to the pre-treatment into the reaction cell R0, in addition to transferring the specimen 22 which has been subjected to the pre-treatment from the cell P0 for pre-treatment to the reaction cell R0, the examination apparatus can be configured to be small as compared with a case where a dispensing mechanism for dispensing the specimen 22 which has not been subjected to the pre-treatment is separately provided.

In the above-described embodiments, as the examination apparatuses 10 and 110, the immunological analysis apparatus that detects the examination target substance included in the specimen by using the antigen-antibody reaction is described as an example. Needless to say, the technology of the present disclosure can be used for an examination apparatus other than the immunological analysis apparatus. In addition, the chemiluminescent immunoassay method has been described as an example of the method of detecting the examination target substance, but the present disclosure is not limited to this method and may be applied to other methods.

In addition, in the above-described embodiments, as a hardware structure of the processor, various processors shown below can be used. Various processors include a programmable logic device (PLD) that is capable of changing a circuit configuration after manufacturing, such as a field-programmable gate array (FPGA), and a dedicated electric circuit that is a processor having a circuit configuration dedicatedly designed for executing specific process, such as an application specific integrated circuit (ASIC), in addition to a CPU that is a general-purpose processor configured to execute software (program) to function as various processing units.

In addition, the above-described process may be executed by one of various processors or may be executed by a combination of two or more processors (for example, a combination of a plurality of FPGAs or a CPU and an FPGA) of the same type or different types. The plurality of processing units may be configured of one processor. As an example in which a plurality of processing units are composed of one processor, there is a form in which a processor that realizes all functions of a system including a plurality of processing units into one integrated circuit (IC) chip is used, such as system-on-chip (SOC).

Furthermore, the hardware structure of the processors is, more specifically, an electric circuit (circuitry) in which circuit elements such as semiconductor elements are combined.

In addition, the technology of the present disclosure is applied to not only the operation program of the examination apparatus but also a non-transitory computer readable storage medium (a USB memory, a digital versatile disc (DVD)-read only memory (ROM), or the like) storing the operation program of the examination apparatus.

The contents described and shown above are detailed descriptions of portions according to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, in order to avoid complication and facilitate understanding of portions according to the technology of the present disclosure, description related to common technical knowledge or the like that does not need to be particularly described for enabling implementation of the technology of the present disclosure is omitted in the contents described and shown above.

The disclosure of Japanese Patent Application No. 2022-091057 filed on June 3, 2022 is incorporated in the present specification by reference. All cited documents, patent applications, and technical standards described in the specification are incorporated by reference in the specification to the same extent as in a case where each individual cited document, patent application, or technical standard is specifically and individually indicated to be incorporated by reference.

## Claims

1. An examination apparatus for performing a measurement process using a cartridge for measurement, having a reaction cell in which a specimen is dispensed and mixed with a reagent, the examination apparatus comprising:
a linear transport passage that transports both cartridges of a cartridge for pre-treatment which is used for a pre-treatment performed on the specimen before the measurement process is executed, the pre-treatment being performed by heating the specimen in a state in which the specimen is mixed with a pre-treatment liquid, and which has a cell for pre-treatment accommodating the specimen and the pre-treatment liquid, and the cartridge for measurement, the transport passage being capable of transporting the cartridge for pre-treatment while performing the pre-treatment on the specimen; and
a specimen transfer mechanism that transfers the pre-treated specimen in which the pre-treatment is completed, from the cartridge for pre-treatment transported at any position between, in the transport passage, a specimen dispensing position where the specimen is dispensed into the reaction cell of the cartridge for measurement and a detection position where an examination target substance in the specimen is optically detected in a downstream of the transport passage in a transport direction, to the cartridge for measurement disposed at the specimen dispensing position.

2. The examination apparatus according to claim 1,
wherein the examination apparatus includes, as the specimen transfer mechanism, a dispensing mechanism that performs suction and discharge of a liquid by a nozzle, and
the dispensing mechanism transfers the pre-treated specimen by suctioning the pre-treated specimen from the cell for pre-treatment of the cartridge for pre-treatment at the any position on the transport passage, and dispensing the pre-treated specimen into the reaction cell of the cartridge for measurement at the specimen dispensing position.

3. The examination apparatus according to claim 2,
wherein the dispensing mechanism is also used for dispensing a specimen which has not been subjected to the pre-treatment into the reaction cell, in addition to the transfer of the pre-treated specimen from the cell for pre-treatment to the reaction cell.

4. The examination apparatus according to claim 2 or 3, further comprising, in a case where a plurality of the cartridges for measurement and a plurality of the cartridges for pre-treatment, which are used for different specimens, are sequentially transported on the transport passage,
a cover member that is capable of covering the cartridges for measurement and the cartridges for pre-treatment above the transport passage and prevents the specimen transferred by the specimen transfer mechanism from being mixed into the cartridge for measurement and the cartridge for pre-treatment, which are used for another specimen.

5. The examination apparatus according to claim 1,
wherein the specimen transfer mechanism includes a dispensing mechanism that performs suction and discharge of a liquid by a nozzle and a pre-treatment cartridge moving mechanism that detaches the cartridge for pre-treatment from the transport passage and moves the cartridge for pre-treatment to an upstream side in the transport direction of the transport passage, and
after the cartridge for pre-treatment is moved by the pre-treatment cartridge moving mechanism, the pre-treated specimen is transferred from the cell for pre-treatment to the reaction cell by the dispensing mechanism.

6. The examination apparatus according to claim 5,
wherein the dispensing mechanism is also used for dispensing a specimen which has not been subjected to the pre-treatment into the reaction cell, in addition to the transfer of the pre-treated specimen from the cell for pre-treatment to the reaction cell.
